# EUROPEAN PATENT APPLICATION

(11) **EP 4 653 461 A1**
(43) Date of publication of application: **26.11.2025**
(21) Application number: 24744825.1
(22) Date of filing: 15.01.2024
(51) Int. Cl.: C07K 16/28, A61P 35/00, A61P 31/00, G01N 33/574, A61K 39/00

(54) **NOVEL ANTI-NKP46 ANTIBODY AND USE THEREOF**

(30) Priority: 16.01.2023 KR 20230006040
(71) Applicant: DE NOVO BIOTHERAPEUTICS CO., LTD., Seoul 03185 (KR)
(72) Inventor: CHOI, Chang Hoon, Seoul 03185 (KR); KIM, Yong Jae, Seoul 03185 (KR); HONG, Mirim, Seoul 03185 (KR); JI, Boram, Seoul 03185 (KR)
(74) Representative: Mewburn Ellis LLP
(86) International application number: PCT/KR2024/000674
(87) International publication number: WO 2024/155044

(57) **Abstract**

The present invention relates to antibodies that specifically bind to NKp46, or antigen-binding fragments thereof, and nucleic acid molecules encoding the same. The antibodies of the present invention bind with high affinity to NKp46, a natural killer cell-specific activation receptor, thereby significantly enhancing the immune activity of NK cells against a variety of pathogenic substances. The antibodies of the present invention may also be useful for providing reliable information on the presence and number of natural killer cells in a biological sample, or for sorting them in a sample with high selectivity.

## Description

### Technical Field

The present invention relates to an antibody or an antigen-binding fragment thereof that specifically binds to NKp46, an activation receptor expressed on natural killer cells, and therapeutic uses thereof. The present invention was supported by the National New Drug Development Project (RS-2023-00258675) of the National Research Foundation for Science and ICT, funded by the Ministry of Science and ICT, Ministry of Trade, Industry and Energy, and Ministry of Health and Welfare.

### Background Art

Cancer immunotherapy has been performed by targeting cancer cells with antibodies specific for cancer cell surface antigens, killing cancer cells by fusion of antigen-presenting cells with cancer cells, or tumor-specific activation of immune cells such as natural killer cells and T cells.

Natural killer cells (NK cells) are lymphoid cells that account for about 10% of blood cells and play a crucial role in the immune response. NK cells perform a variety of biological functions, but their ability to remove cancer cells or the cells infected with exogenous pathogens is particularly important in eliminating abnormal cells that have the potential to become pathological.

Most NK cells present in the body exist in an inactivated state under normal conditions. However, since activated NK cells are required for therapeutic applications, active research is being conducted to effectively activate NK cells derived from healthy or patient blood.

The three activation receptors found on NK cells, NKp30, NKp44, and NKp46, are natural cytotoxicity receptors (NCRs) that play an important role in the antitumor and antiviral defenses of NK cells. NKp46 is expressed only by NK cells, making it not only an important activation receptor but also an important marker for identifying, isolating, or detecting NK cells. Its ligands range from viral ligands such as hemagglutinin (HA) and hemagglutinin-neuraminidase (HN) from influenza virus, Sendai virus, Newcastle disease virus, and poxvirus, to bacterial ligands such as Fusobacterium nucleatum, and to ligands expressed by tumor, adipocyte, and human pancreatic beta cells.

Therefore, there is a growing need for the development of antibodies that specifically recognize NKp46, as they can be used to enhance the immune activity of NK cells against various pathogenic substances and can be useful for the specific detection and isolation of NK cells from mixtures containing non-homogeneous cell populations.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop an efficient anti-NKp46 antibodies that bind with high affinity to NKp46, an activating receptor expressed on natural killer (NK) cells, promote the activation of endogenous or exogenously administered NK cells, enhance the *ex vivo* expansion of NK cells, and thereby induce a synergistic antitumor immune response. As a result, the present inventors have successfully identified a number of antibodies that recognize NKp46 with remarkable affinity and specificity, and elucidated the structure of their antigen-binding regions, thereby completing the present invention.

Accordingly, it is an object of the present invention to provide an antibody or an antigen-binding fragment thereof that specifically binds to NKp46 and a nucleic acid molecule encoding the same.

It is another object of the present invention to provide a composition for preventing or treating a cancer or an infectious disease comprising said antibody or antigen-binding fragment thereof, or nucleic acid molecules encoding the same them as active ingredients; a composition for activating immune cells; a composition for the detecting NKp46 expressing cells; and a composition for the diagnosing a cancer.

Other objects and advantages of the present invention will become more apparent from the following detailed description, the appended claims, and the accompanying drawings.

### Technical Solution

In one aspect of this invention, there is provided an antibody or an antigen-binding fragment thereof that specifically binds to NKp46 comprising a heavy chain variable region comprising an HCDR1 having the amino acid sequence selected from the group consisting of SEQ ID NOs: 33 to 38; an HCDR2 having the amino acid sequence region selected from the group consisting of SEQ ID NOs: 39 to 47; and an HCDR3 having the amino acid sequence selected from the group consisting of SEQ ID NOs: 48 to 56.

The present inventors have made intensive studies to develop an efficient anti-NKp46 antibodies that bind with high affinity to NKp46, an activating receptor expressed on natural killer (NK) cells, promote the activation of endogenous or exogenously administered NK cells, enhance the ex vivo expansion of NK cells, and thereby induce a synergistic antitumor immune response. As a result, a number of antibodies that recognize NKp46 with remarkable affinity and specificity have been identified along with the structure of their antigen recognition sites.

As used herein, the term "antibody" refers to a peptide molecule that that specifically recognizes and binds to a specific epitope of NKp46, and includes antigen-binding fragments of the antibody (immunologically active fragments) as well as the whole antibody molecule. A complete antibody has a structure with two full-length light chains and two full-length heavy chains, which are linked to each other by a disulfide bond. The heavy chain constant region has the gamma (γ), mu (µ), alpha (α), delta (δ), and epsilon (ε) types and is subclassified into gammal (γ1), gamma2 (γ2), gamma3 (γ3), gamma4 (γ4), alphal (α1), and alpha2 (α2). The light chain constant region has kappa (κ) and lambda (λ) types.

As used herein, the term "antigen-binding fragment of the antibody" refers to a portion of a polypeptide that has significant antigen-antibody binding functionality within the overall structure of an immunoglobulin. Examples of the antigen-binding fragment include, but are not limited to, Fab, F(ab'), F(ab')2, Fv, and nanobody (sybody).

Among antibody fragments, Fab is a structure with one antigen-binding site containing variable regions of the light and heavy chains, a constant region of the light chain and the first constant region of the heavy chain (CH1).

Fab' differs from Fab in that it has a hinge domain containing one or more cysteine residues at the C-terminus of the heavy chain CH1 domain. F(ab')2 antibodies are generated by disulfide bond formed by cysteine residues in the hinge of Fab'. Recombinant techniques for generating Fv fragments with minimal antibody fragments that have only a heavy chain variable region and a light chain variable region are disclosed in WO88/10649, WO88/106630, WO88/07085, WO88/07086, and WO88/09344. Two-chain Fv has a heavy chain variable region and a light chain variable region linked by non-covalent bonds, while single-chain Fv has a heavy chain variable region and a light chain variable region linked by covalent bonds, usually via a peptide linker, or directly at the C-terminus, which can form a dimer-like structure.

As used herein, the term "light chain" refers to both full-length light chain and fragments thereof comprising a variable region VL and a constant region CL with the amino acid sequence having sufficient variable region sequence to confer specificity to an antigen.

As used herein, the term "heavy chain" refers to both full-length heavy chain and fragments thereof comprising a variable region VH and three constant regions CH1, CH2, and CH3 with the amino acid sequence having sufficient variable region sequence to confer specificity to an antigen.

As used herein, the term "complementarity determining region (CDR)" refers to the amino acid sequence of the hypervariable region of the heavy and light chains of an immunoglobulin. The heavy chain (HCDR1, HCDR2, and HCDR3) and light chain (LCDR1, LCDR2, and LCDR3) each contain three CDRs, which provide the primary contact residues for an antibody to bind to an antigen or epitope.

The scope of the antibody or antigen-binding fragment of the present invention includes variants having conservative amino acid substitutions in the CDR region. Furthermore, the antibody or antigen-binding fragment of the present invention includes variants of the amino acid in the attached sequence listings, to the extent that they are capable of specifically recognizing NKp46. For example, additional changes can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

In addition, amino acid substitutions in proteins that do not alter the activity of the molecule as a whole are known in the art (H. Neurath, R.L. Hill, The Proteins, Academic Press, New York, 1979). The most common exchanges are between the amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly.

Considering biologically equivalent variations, the amino acid sequence constituting the antibody of the present invention may encompass sequences having substantial identity to them. Sequences having the substantial identity show at least 61%, specifically at least 70%, more specifically at least 80%, even more specifically at least 90% similarity to the amino acid of this invention, as measured using one of the sequence comparison algorithms. Methods of alignment of sequences for comparison are well-known in the art. Various methods and algorithms for alignment are disclosed in Huang et al., Comp. Appl. BioSci. 8:155-65(1992) and Pearson et al., Meth. Mol. Biol. 24:307-31(1994).

According to a specific embodiment, the antibody or the antigen-binding fragment thereof further comprises a light chain variable region comprising an LCDR1 having the amino acid sequence selected from the group consisting of SEQ ID NOs: 1 to 6; an LCDR2 having the amino acid sequence selected from the group consisting of sequences SEQ ID NOs: 7 to 12; and LCDR3 having the amino acid sequence selected from the group consisting of SEQ ID NOs: 13 to 19:

According to a specific embodiment, the antibody or the antigen-binding fragment thereof is selected from the group consisting of (a) to (m) as set forth below:
(a) an antibody or antigen-binding fragment thereof comprising a light chain variable region comprising LCDR1 having the amino acid sequence of SEQ ID NO: 1, LCDR2 having the amino acid sequence of SEQ ID NO: 7, and LCDR3 having the amino acid sequence of SEQ ID NO: 13; and a heavy chain variable region comprising HCDR1 having the amino acid sequence of SEQ ID NO: 33, HCDR2 having the amino acid sequence of SEQ ID NO: 39, and HCDR3 having the amino acid sequence of SEQ ID NO: 48;
(b) an antibody or antigen-binding fragment thereof comprising a light chain variable region comprising LCDR1 having the amino acid sequence of SEQ ID NO: 2, LCDR2 having the amino acid sequence of SEQ ID NO: 8, and LCDR3 having the amino acid sequence of SEQ ID NO: 14; and a heavy chain variable region comprising HCDR1 having the amino acid sequence of SEQ ID NO: 34, HCDR2 having the amino acid sequence of SEQ ID NO: 40, and HCDR3 having the amino acid sequence of SEQ ID NO: 49;
(c) an antibody or antigen-binding fragment thereof comprising a light chain variable region comprising LCDR1 having the amino acid sequence of SEQ ID NO: 3, LCDR2 having the amino acid sequence of SEQ ID NO: 9, and LCDR3 having the amino acid sequence of SEQ ID NO: 15; and a heavy chain variable region comprising HCDR1 having the amino acid sequence of SEQ ID NO: 35, HCDR2 having the amino acid sequence of SEQ ID NO: 41, and HCDR3 having the amino acid sequence of SEQ ID NO: 50;
(d) an antibody or antigen-binding fragment thereof comprising a light chain variable region comprising LCDR1 having the amino acid sequence of SEQ ID NO: 4, LCDR2 having the amino acid sequence of SEQ ID NO: 10, and LCDR3 having the amino acid sequence of SEQ ID NO: 16; and a heavy chain variable region comprising HCDR1 having the amino acid sequence of SEQ ID NO: 35, HCDR2 having the amino acid sequence of SEQ ID NO: 42, and HCDR3 having the amino acid sequence of SEQ ID NO: 51;
(e) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 17; and a heavy chain variable region comprising HCDR1 having the amino acid sequence of SEQ ID NO: 35, HCDR2 having the amino acid sequence of SEQ ID NO: 42, and HCDR3 having the amino acid sequence of SEQ ID NO: 52;
(f) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 18; and a heavy chain variable region comprising HCDR1 having the amino acid sequence of SEQ ID NO: 35, HCDR2 having the amino acid sequence of SEQ ID NO: 43, and HCDR3 having the amino acid sequence of SEQ ID NO: 52;
(g) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 17; and a heavy chain variable region comprising HCDR1 having the amino acid sequence of SEQ ID NO: 36, HCDR2 having the amino acid sequence of SEQ ID NO: 44, and HCDR3 having the amino acid sequence of SEQ ID NO: 53;
(h) an antibody or antigen-binding fragment thereof comprising a light chain variable region comprising LCDR1 having the amino acid sequence of SEQ ID NO: 4, LCDR2 having the amino acid sequence of SEQ ID NO: 10, and LCDR3 having the amino acid sequence of SEQ ID NO: 19; and a heavy chain variable region comprising HCDR1 having the amino acid sequence of SEQ ID NO: 37, HCDR2 having the amino acid sequence of SEQ ID NO: 45, and HCDR3 having the amino acid sequence of SEQ ID NO: 54;
(i) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 17; and a heavy chain variable region comprising HCDR1 having the amino acid sequence of SEQ ID NO: 38, HCDR2 having the amino acid sequence of SEQ ID NO: 42, and HCDR3 having the amino acid sequence of SEQ ID NO: 55;
(j) an antibody or antigen-binding fragment thereof comprising a light chain variable region comprising LCDR1 having the amino acid sequence of SEQ ID NO: 6, LCDR2 having the amino acid sequence of SEQ ID NO: 11, and LCDR3 having the amino acid sequence of SEQ ID NO: 18; and a heavy chain variable region comprising HCDR1 having the amino acid sequence of SEQ ID NO: 35, HCDR2 having the amino acid sequence of SEQ ID NO: 43, and HCDR3 having the amino acid sequence of SEQ ID NO: 52;
(k) an antibody or antigen-binding fragment thereof comprising a light chain variable region comprising LCDR1 having the amino acid sequence of SEQ ID NO: 1, LCDR2 having the amino acid sequence of SEQ ID NO: 7, and LCDR3 having the amino acid sequence of SEQ ID NO: 13; and a heavy chain variable region comprising HCDR1 having the amino acid sequence of SEQ ID NO: 33, HCDR2 having the amino acid sequence of SEQ ID NO: 46, and HCDR3 having the amino acid sequence of SEQ ID NO: 48;
(l) an antibody or antigen-binding fragment thereof comprising a light chain variable region comprising LCDR1 having the amino acid sequence of SEQ ID NO:5, LCDR2 having the amino acid sequence of SEQ ID NO:12, and LCDR3 having the amino acid sequence of SEQ ID NO:17 of SEQ ID NO: and a heavy chain variable region comprising having the amino acid sequence of SEQ ID NO: 36, HCDR2 having the amino acid sequence of SEQ ID NO: 44, and HCDR3 having the amino acid sequence of SEQ ID NO: 53; and
(m) an antibody or antigen-binding fragment thereof comprising a light chain variable region comprising LCDR1 having the amino acid sequence of SEQ ID NO: 4, LCDR2 having the amino acid sequence of SEQ ID NO: 10, and LCDR3 having the amino acid sequence of SEQ ID NO: 19; and a heavy chain variable region comprising HCDR1 having the amino acid sequence of SEQ ID NO: 37, HCDR2 having the amino acid sequence of SEQ ID NO: 47, and HCDR3 having the amino acid sequence of SEQ ID NO: 56.

More specifically, said (a) comprises a light chain variable region having the amino acid sequence of SEQ ID NO: 40 and a heavy chain variable region having the amino acid sequence of SEQ ID NO: 92. Most specifically, said (a) comprises the amino acid sequence of SEQ ID NO: 105.

More specifically, said (b) comprises a light chain variable region having the amino acid sequence of SEQ ID NO: 41 and a heavy chain variable region having the amino acid sequence of SEQ ID NO: 93. Most specifically, said (a) comprises the amino acid sequence of SEQ ID NO: 106.

More specifically, said (c) comprises a light chain variable region having the amino acid sequence of SEQ ID NO: 42 and a heavy chain variable region having the amino acid sequence of SEQ ID NO: 94. Most specifically, said (a) comprises the amino acid sequence of SEQ ID NO: 107.

More specifically, said (d) comprises a light chain variable region having the amino acid sequence of SEQ ID NO: 43 and a heavy chain variable region having the amino acid sequence of SEQ ID NO: 95. Most specifically, said (a) comprises the amino acid sequence of SEQ ID NO: 108.

More specifically, said (e) comprises a light chain variable region having the amino acid sequence of SEQ ID NO: 44 and a heavy chain variable region having the amino acid sequence of SEQ ID NO: 96. Most specifically, said (a) comprises the amino acid sequence of SEQ ID NO: 109.

More specifically, said (f) comprises a light chain variable region having the amino acid sequence of SEQ ID NO: 45 and a heavy chain variable region having the amino acid sequence of SEQ ID NO: 97. Most specifically, said (a) comprises the amino acid sequence of SEQ ID NO: 110.

More specifically, said (g) comprises a light chain variable region having the amino acid sequence of SEQ ID NO: 46 and a heavy chain variable region having the amino acid sequence of SEQ ID NO: 98. Most specifically, said (a) comprises the amino acid sequence of SEQ ID NO: 111.

More specifically, said (h) comprises a light chain variable region having the amino acid sequence of SEQ ID NO: 47 and a heavy chain variable region having the amino acid sequence of SEQ ID NO: 99. Most specifically, said (a) comprises the amino acid sequence of SEQ ID NO: 112.

More specifically, said (i) comprises a light chain variable region having the amino acid sequence of SEQ ID NO: 48 and a heavy chain variable region having the amino acid sequence of SEQ ID NO: 100. Most specifically, said (a) comprises the amino acid sequence of SEQ ID NO: 113.

More specifically, said (j) comprises a light chain variable region having the amino acid sequence of SEQ ID NO: 49 and a heavy chain variable region having the amino acid sequence of SEQ ID NO: 101. Most specifically, said (a) comprises the amino acid sequence of SEQ ID NO: 114.

More specifically, said (k) comprises a light chain variable region having the amino acid sequence of SEQ ID NO: 50 and a heavy chain variable region having the amino acid sequence of SEQ ID NO: 102. Most specifically, said (a) comprises the amino acid sequence of SEQ ID NO: 115.

More specifically, said (1) comprises a light chain variable region having the amino acid sequence of SEQ ID NO: 51 and a heavy chain variable region having the amino acid sequence of SEQ ID NO: 103. Most specifically, said (a) comprises the amino acid sequence of SEQ ID NO: 116.

More specifically, said (m) comprises a light chain variable region having the amino acid sequence of SEQ ID NO: 52 and a heavy chain variable region having the amino acid sequence of SEQ ID NO: 104. Most specifically, said (a) comprises the amino acid sequence of SEQ ID NO: 117.

In another aspect of this invention, there is provided a nucleic acid molecule encoding the antibody or the antigen-binding fragment of the present invention that specifically binds to NKp46.

As used herein, the term "nucleic acid molecule" is meant to encompass DNA (gDNA and cDNA) and RNA molecules. Nucleotides, which are the basic structural units in nucleic acid molecules, include not only natural nucleotides, but also analogues having modified sugar or base moieties (Uhlman and Peyman, Chemical Reviews, 90:543-584

(1990)). It will be clear to those skilled in the art that the nucleotide sequence encoding the amino acid sequence of a full-length antibody or a heavy chain, light chain variable region thereof, may involve modifications including additions, deletions, or non-conservative or conservative substitutions of nucleotides.

The nucleic acid molecule of the present invention is interpreted to include a nucleotide sequence that is substantially identical to any of the above nucleotide sequences. Substantial identity means a sequence showing at least 80% homology, specifically at least 90% homology, more specifically at least 95% homology as determined by aligning the sequence of the present invention with any other sequence to correspond to each other as much as possible and analyzing the aligned sequence using an algorithm commonly used in the art.

According to a specific embodiment, the nucleic acid molecule used in the present invention may be an mRNA molecule, more specifically an in vitro transcribed (IVT) mRNA.

When mRNA is used as the nucleic acid molecule of the invention, various modifications can be made to improve the efficiency of expression (translation) of the antibody or the antigen-binding fragment, including, for example, changes in the length of the poly(A) tail or substitution of some adenine bases; modification of the 5'cap; application of one or more modified nucleosides. The modified nucleosides include, for example, N1-methylpseudouridine, pseudouridine, 2-thiouridine, 5-methyluridine, or 5-methylcytidine, 5-methylcytidine and 5-methoxyuridine, but are not limited thereto. Any modified nucleoside known in the art to reduce the immunogenicity of an mRNA molecule may also be used.

In still another aspect of this invention, there is provided a gene delivery vehicle comprising said nucleic acid molecule.

According to the present invention, the antibody or the antigen-binding fragment thereof of the present invention may be obtained recombinantly by expressing a nucleic acid molecule encoding it in a host cell.

As used herein, the term "express" refers to being artificially replicated as an extrachromosomal factor or by chromosomal integration in a target cell via a gene delivery system to cause the target cell to express an exogenous gene or overexpress an endogenous gene. Accordingly, "express" may be used interchangeably with "transformation", "transfection", or "transduction". More specifically, "to express" in the present invention refers to cause a target cell to artificially express an exogenous gene.

The term "gene delivery system" or "gene delivery vehicle" as used herein refers to any means for delivering a gene into a cell, and the term "gene delivery" has the same meaning as intracellular transduction of the gene. At the cell or tissue level, gene delivery has the same meaning as spread of the gene. Thus, the gene delivery system of the present invention may be referred to as a gene transduction system or a gene spread system.

The gene delivery vehicle of the present invention may comprise an expression cassette, which is a polynucleotide construct containing all the elements necessary for the autonomous expression of the gene to be introduced. The expression cassette typically includes a promoter, a transcription termination signal, a ribosome binding site, and a translation termination signal that is operatively linked to the gene. The expression cassette may be in the form of a self-replicating expression vector. As used herein, the term "operatively linked" refers to a functional linkage between a nucleic acid expression regulatory sequence (e.g., a promoter, a signal sequence, or an array of transcription regulation factor binding sites) and the target nucleic acid sequence. Through the linkage, the regulatory sequence regulates the transcription and/or translation of the target nucleic acid sequence.

The recombinant vector systems of the present invention can be constructed by a variety of methods known in the art, and specific methods are disclosed in Sambrook et al., Molecular Cloning, A Laboratory Manual, Cold Spring Harbor Laboratory Press (2001).

The vectors of the present invention can typically be constructed as cloning vectors or expression vectors. Furthermore, the vectors of the invention can be constructed in prokaryotic or eukaryotic cells as hosts. For example, if the vectors of the invention are expression vectors using prokaryotic cells as hosts, a strong promoter capable of driving transcription (e.g., tac promoter, lac promoter, lacUV5 promoter, 1pp promoter, pLλ promoter, pRλ promoter, pRλ promoter, rac5 promoter, amp promoter, recA promoter, SP6 promoter, trp promoter, and T7 promoter, among others), a ribosome binding site for initiation of detoxification, and a transcription/detoxification termination sequence are generally contained. When *E. coli* (e.g., HB101, BL21, DH5α, etc.) is used as the host cell, the promoter and operator sites of the *E. coli* tryptophan biosynthetic pathway (Yanofsky, C. J. Bacteriol. 158:1018-1024 (1984)) and the left-handed promoter of phage λ (pLλ promoter, Herskowitz, I. and Hagen, D. Ann. Rev. Genet. 14:399-445 (1980)) can be utilized as regulatory sites. If Bacillus is utilized as the host cell, the promoter of the toxin protein gene of *Bacillus churrigensis* (Appl. Environ. Microbiol. 64:3932-3938 (1998); Mol. Gen. Genet. 250:734-741

(1996)) or any promoter that is expressible in *Bacillus* can be used as a regulatory site.

The recombinant vectors of the present invention can be constructed by manipulating plasmids (e.g., pCL, pSC101, pGV1106, pACYC177, Co1E1, pKT230, pME290, pBR322, pUC8/9, pUC6, pBD9, pHC79, pIJ61, pLAFR1, pHV14, pGEX series, pET series, and pUC19, etc.), phages (e.g.: λgt4-λB, λ-Charon, λΔz1, and M13, etc.), or viruses (e.g., SV40, etc.).

On the other hand, in case the vector of the present invention is an expression vector and is hosted in a eukaryotic cell, it may comprise a promoter derived from the genome of a mammalian cell (e.g., metallothionein promoter, β-actin promoter, human hemoglobin promoter, and human muscle creatine promoter) or a mammalian virus (e.g., adenovirus late promoter, vaccinia virus 7.5K promoter, Vaccinia virus 7. 5K promoter, SV40 promoter, cytomegalovirus (CMV) promoter, tk promoter of HSV, mouse mammary tumor virus (MMTV) promoter, LTR promoter of HIV, Moloney virus promoter Epstein-Barr virus (EBV) promoter and Roos Sakoma virus (RSV) promoter). These vectors typically have a polyadenylation sequence as a transcription termination sequence.

The recombinant vectors of the present invention may also be fused with other sequences to facilitate purification of antibodies expressed therefrom. The fused sequences may be, for example, glutathione S-transferase (Pharmacia, USA); maltose binding protein (NEB, USA); FLAG (IBI, USA); tag sequences such as 6x His (hexahistidine; Quiagen, USA), Pre-S1, c-Myc; and leader sequences such as OmpA, PelB. Furthermore, since the protein expressed by the vectors of the present invention is an antibody, the expressed antibody can be easily purified through a protein A column or the like without the need for additional sequences for purification.

The recombinant vectors of the present invention can also include antibiotic resistance genes conventionally utilized in the art as selection markers, for example, resistance genes for ampicillin, gentamicin, carbenicillin, chloramphenicol, streptomycin, kanamycin, geneticin, neomycin, and tetracycline.

The vectors expressing the antibodies of the invention can be either a vector system in which the light and heavy chains are expressed simultaneously in a single vector, or a system in which the light and heavy chains are expressed in separate vectors. In the latter case, the two vectors are introduced into the host cell by co-transformation and targeted transformation. Co-transformation involves simultaneous introduction of the respective vector DNA encoding the light and heavy chains into the host cell, followed by selection of cells expressing both light and heavy chains. Targeted transfection is a method of selecting cells transformed with a vector containing the light chain (or heavy chain), and the selected cells expressing the light chain are transformed again with a vector containing the heavy chain (or light chain) to finally select cells expressing both the light and heavy chains.

The gene delivery vehicles of the present invention can be any gene delivery system conventionally utilized for gene insertion, including, but not limited to, plasmids, adenoviruses, adeno-associated viruses (AAVs), retroviruses, lentiviruses, herpes simplex viruses, bacilliviruses, liposomes, niosomes, and lipid nanoparticles.

In still another aspect of this invention, there is provided a host cell transformed with the gene delivery vehicle of the present invention.

In one embodiment, the host cell is a cell transformed with a recombinant vector containing a gene encoding an antibody of the invention. Any host cell known in the art can be used to stably and continuously clone and express the vectors of the invention. For example, in the case of prokaryotic cells, the host cell includes, but is not limited to, *Escherichia coli ,* the strains of the genus *Bacillus* such as *Bacillus subtilis* and *Bacillus thuringiensis, Streptomyces, Pseudomonas* (e.g., *Pseudomonas putida*), *Proteus mirabilis* and or *Staphylococcus* (e.g., *Staphylococcus carnosus*)*.*

The suitable eukaryotic host cells that may be used in the present invention include, for example, fungi such as *Aspergillus* species, yeasts such as *Pichia pastoris, Saccharomyces cerevisiae, Schizosaccharomyces,* and *Neurospora crassa,* other lower eukaryotic cells, cells from higher eukaryotic organisms such as insect-derived cells, and cells derived from plants or mammals.

As used herein, the term "transfection" refers to the introduction of a target gene into a host cell using a gene delivery vehicle of the present invention and includes any method of introducing a nucleic acid into an organism, cell, tissue or organ. Transfection can be performed by selecting a standard technique suitable for the host cell as known in the art. These methods include, but are not limited to, electroporation, protoplast fusion, calcium phosphate (CaPO₄) precipitation, calcium chloride (CaCl₂) precipitation, agitation with silicon carbide fibers, agrobacterium-mediated transfection, PEG, dextran sulfate, lipofectamine, and desiccation/inhibition-mediated transfection.

The culture of host cells for the production of antibodies or antigen-binding fragments thereof can be accomplished using any suitable medium and culture conditions known in the art. These culture procedures can be easily adapted by those skilled in the art to the strain selected, and examples of specific culture methods are disclosed in the literatures (e.g., James M. Lee, Biochemical Engineering, Prentice-Hall International Editions, 138-176). Antibodies obtained by host cell culture can be used in an un-purified state or can be further purified using a variety of conventional methods, e.g., dialysis, salt precipitation, and chromatography. When chromatography is used, the type and sequence of columns can be selected from ion-exchange chromatography, size-exclusion chromatography, affinity chromatography, etc. depending on the nature of the antibody and the method of host cell culture.

In still another aspect of this invention, there is provided a composition for preventing or treating a cancer or an infectious disease comprising, as an active ingredient, the antibody or the antigen-binding fragment thereof, the nucleic acid, or the gene delivery vehicle of the present invention.

In still another aspect of this invention, there is provided a method for preventing or treating a cancer or an infectious disease comprising administering the antibody or the antigen-binding fragment thereof, the nucleic acid, or the gene delivery vehicle of the present invention to a subject in need thereof.

As used herein, the term "preventing" refers to inhibiting the occurrence of a disorder or disease in a subject who has never been diagnosed as having the disorder or disease, but is at risk of developing the condition or disease.

As used herein, the term "treating" refers to (a) inhibiting the progress of a disorder, disease or symptom; (b) alleviating the disorder, disease or symptom; or (c) eliminating the disorder, disease or symptom. The compositions of the present invention, when administered to a subject, significantly enhance the immune response to a tumor or pathogenic strain by inducing the activation of endogenous NK cells or exogenous autologous/allogeneic NK cells infused as therapeutic cells, thereby inhibiting the progression of the disease, eliminating or alleviating the symptoms caused by the disease. Therefore, the composition of the present invention may itself be a cell therapy composition, or may be applied as a therapeutic adjuvant for the disease by being administered together with other active ingredients. Accordingly, the term "treatment" or "therapeutic agent" in the present specification includes the meaning of "therapeutic aid" or "therapeutic adjuvant".

As used herein, the term "administer" or "administration" refers to the direct administration of a therapeutically effective amount of the composition of the present invention to a subject so that an equal amount is formed in the body of the subject.

As used herein, the terms "therapeutically effective amount" refer to the content of the composition of the present invention that is sufficient to provide a therapeutic or prophylactic effect to a subject to whom the composition is to be administered, and thus include the meaning of a "prophylactically effective amount".

As used herein, the term "subject" includes, without limitation, humans, mice, rats, guinea pigs, dogs, cats, horses, cows, pigs, monkeys, chimpanzees, baboons or rhesus monkeys. Specifically, the subject of the present invention is humans.

The compositions of the invention efficiently activate immune cells, specifically NK cells, and can therefore be applied to the treatment of tumors and infectious diseases. The antibodies of the present invention can be applied to all types of tumors, including solid and hematologic cancers. Solid cancers refer to cancers that form masses in organs, unlike hematologic cancers, and include most cancers that occur in organs. The cancers treatable using the antibody of the present invention may include, but are not limited to, gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumors, neuroblastoma, retinoblastoma, head and neck cancer, salivary gland cancer, and lymphoma. Infectious diseases that can be treated by immune cells activated by the antibody of the present invention are diseases caused by infections with viruses or pathogens, and encompass all diseases that can be transmitted and contracted through the respiratory tract, blood, skin contact, and other routes. Such infectious diseases include, for example, hepatitis B and C, human papillomavirus (HPV) infection, cytomegalovirus infection, viral respiratory diseases, and influenza, but are not limited thereto

According to a specific embodiment, the composition further comprises immune cells.

As used herein, the term "immune cell" refers to any cell involved in the initiation or promotion of an immune response, and more specifically, an immune effector cell. Immune cells include, for example, but are not limited to, T cells, B cells, natural killer (NK) cells, natural killer T (NKT) cells, and mast cells. More specifically, said immune cells are natural killer cells.

In still another aspect of this invention, there is provided a composition for activating immune cells comprising, as an active ingredient, the antibody or the antigen-binding fragment thereof, the nucleic acid, or the gene delivery vehicle of the present invention.

In still another aspect of this invention, there is provided a method for activating immune cells comprising administering the antibody or the antigen-binding fragment thereof, the nucleic acid, or the gene delivery vehicle of the present invention to a subject in need thereof.

In still another aspect of this invention, there is provided a composition for detecting NKp46 expressing cells comprising the antibody or the antigen-binding fragment thereof of the present invention.

In still another aspect of this invention, there is provided a method for detecting NKp46 expressing cells comprising contacting the antibody or the antigen-binding fragment thereof of the present invention with a sample to be analyzed.

According to the present invention, the antibodies of the present invention specifically recognize NKp46 which is exclusively expressed on natural killer cells, therefore may be used to selectively isolate, detect and quantify natural killer cells in biological samples containing various cell populations. The term "detection" is therefore used interchangeably with "isolation", "sorting" and "quantification".

Detection of NKp46 expressing cells in a biological sample can be performed by detection of antigen-antibody complex formation using a colorimetric method, electrochemical method, fluorimetric method, luminometry, particle counting method, visual assessment, or scintillation counting method. "Detecting" as used herein refers to detecting antigen-antibody complexes which can be performed using a variety of labeling agents. Specific examples of labeling agents include enzymes, fluorophores, ligands, luminescent agents, microparticles, or radioisotopes.

Enzymes used as detection markers include acetylcholinesterase, alkaline phosphatase, β-D-galactosidase, horseradish peroxidase, and β-latamase, and fluorophores include fluorescein, Eu(3+), Eu(3+) chelates or cryptates, Ligands include biotin derivatives; luminescent agents include acridinium esters and isorhamnol derivatives; microparticles include colloidal gold and colored latex; radioisotopes include ⁵⁷Co, ³H, ¹²⁵I, and ¹²⁵I-Bonton Hunter reagents; and radioactive isotopes include ⁵⁷Co, ³H, ¹²⁵I-Bonton Hunter reagents.

According to one embodiment, antigen-antibody complexes can be detected using enzyme-linked immunosorbent assays (ELISA). Enzyme-linked immunosorbent assays include direct ELISA using a labeled antibody that recognizes an antigen attached to a solid support, and indirect ELISA using a labeled secondary antibody that recognizes a capture antibody in a complex of an antibody that recognizes an antigen attached to a solid support, a direct sandwich ELISA using another labeled antibody that recognizes the antigen in a complex with the antibody conjugated to the solid support, and an indirect sandwich ELISA using a labeled secondary antibody that recognizes the antigen in a complex with the antibody conjugated to the solid support, followed by reaction with another antibody that recognizes the antigen in a complex with the antibody conjugated to the solid support. The antibodies of the present invention may have a detection label, and if not, the antibodies of the present invention may be identified by processing with another antibody that can capture the antibody and has a detection label.

As used herein, the term "biological sample" refers to any sample containing cells expressing NKp46 that is obtained from a mammal, including a human, and includes, but is not limited to, tissue, cells, whole blood, serum, plasma, saliva, urine, lymphatic fluid, spinal fluid, tissue autopsy specimens (brain, skin, lymph nodes, spinal cord, etc.), cell culture supernatants, ruptured eukaryotic cells, and bacterial expression systems. More specifically, the sample is tissue, cells, whole blood, serum, plasma, saliva, urine, lymphatic fluid, or spinal fluid, and more specifically, whole blood, serum, or plasma.

In still another aspect of this invention, there is provided a composition for diagnosing a cancer comprising the antibody or the antigen-binding fragment thereof of the present invention as an active ingredient.

In still another aspect of this invention, there is provided a method for diagnosing a cancer comprising administering the antibody or the antigen-binding fragment thereof of the present invention to a subject in need thereof.

As used herein, the term "diagnosis" includes a determination of susceptibility of a subject to a particular disease, a determination of whether a subject currently has a particular disease, and a determination of the prognosis of a subject with a particular disease.

As used herein, the term "a composition for diagnosing" refers to an integrated mixture or device comprising a means of measuring the expression of NKp46 protein to determine the presence of, or predict the likelihood of, a pathological condition associated with the expression status of NKp46, such as cancer, and may also be expressed as a "diagnostic kit".

### Advantageous Effects

The features and advantages of the present invention are summarized as follows:
(a) The present invention provides antibodies that specifically bind to NKp46, or antigen-binding fragments thereof, and nucleic acid molecules encoding the same.
(b) The antibodies of the present invention bind with high affinity to NKp46, a natural killer cell-specific activation receptor, thereby significantly enhancing the immune activity of NK cells against a variety of pathogenic substances. The antibodies of the present invention may also be useful for providing reliable information on the presence and number of natural killer cells in a biological sample, or for sorting them in a sample with high selectivity.

### Brief Description of Drawings

FIG. 1 shows the results of an ELISA confirming the binding of antibodies obtained from sera of mice immunized with NKp46 antigen, to human NKp46 protein and monkey NKp46 protein.

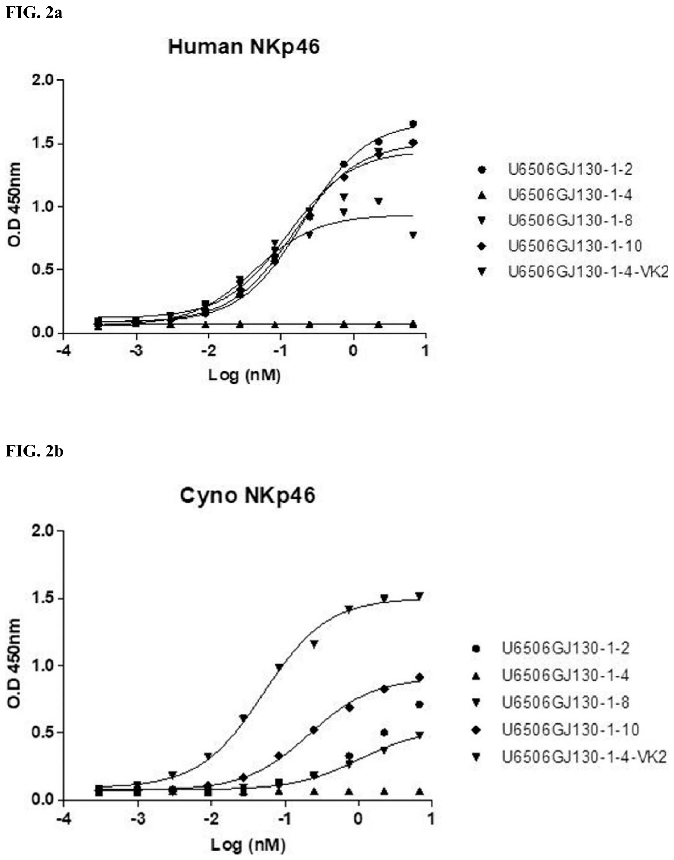

FIG. 2 shows the results of ELISA confirming the binding between five mouse monoclonal antibodies screened by the beacon method and the human NKp46 protein (FIG. 2a) and monkey NKp46 protein (FIG. 2b), respectively.
FIG. 3 shows the results of SDS-PAGE analysis used to verify the size and purity of five mouse monoclonal antibodies, selected by the Beacon method and produced as mouse/human chimeric IgG4 antibodies.
FIGS. 4a to 4b illustrate flow cytometric analysis of binding between five mouse monoclonal antibodies selected by the beacon method and human NKp46-expressing CHO-K1 cells.
FIG. 5 shows the binding of eight mouse monoclonal antibodies selected by the hybridoma method to human NKp46 protein (FIG. 5a) and monkey NKp46 protein (FIG. 5b) by ELISA, respectively.
FIGS. 6a to 6b shows the results of SDS-PAGE analysis used to verify the size and purity of eight mouse monoclonal antibodies, selected by the hybridoma method and produced as mouse/human chimeric IgG4 antibodies."
FIGS. 7a to 7c illustrate the results of flow cytometric analysis of binding between eight mouse monoclonal antibodies selected by the beacon method and human NKp46-expressing CHO-K1 cells.
FIG 8 is a schematic representation of a surface plasmon resonance (SPR) procedure for measuring the binding of an anti-NKp46 antibody to human NKp46 protein.
FIGS 9a to 9b show surface plasmon resonance (SPR) results for binding of 10 mouse monoclonal antibodies to the human NKp46 protein.
FIG. 10 is the result of surface plasmon resonance (SPR) showing the binding of three humanized antibodies to the human NKp46 protein.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Example

### Example 1: Screening of anti-NKp46 antibodies

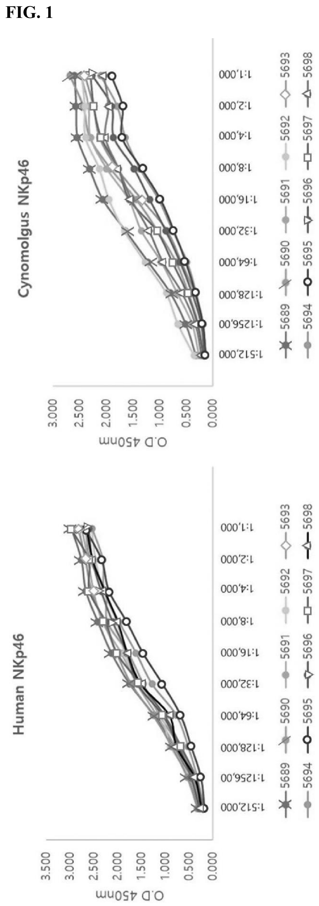

After confirming by ELISA that antibodies against human and monkey NKp46 were generated after immunizing mice with human NKp46 antigen (FIG. 1), plasma cells in the spleen of mice were isolated using a CD138 kit and screened for antibodies that specifically bind to human and monkey NKp46 by two screening methods: beacon screening and hybridoma screening. First, isolated plasma cells that specifically bind to human NKp46 and monkey NKp46 were screened using beacon equipment, and then the sequences of antibodies that bind to NKp46 were obtained by sequence analysis of the mouse variable region.

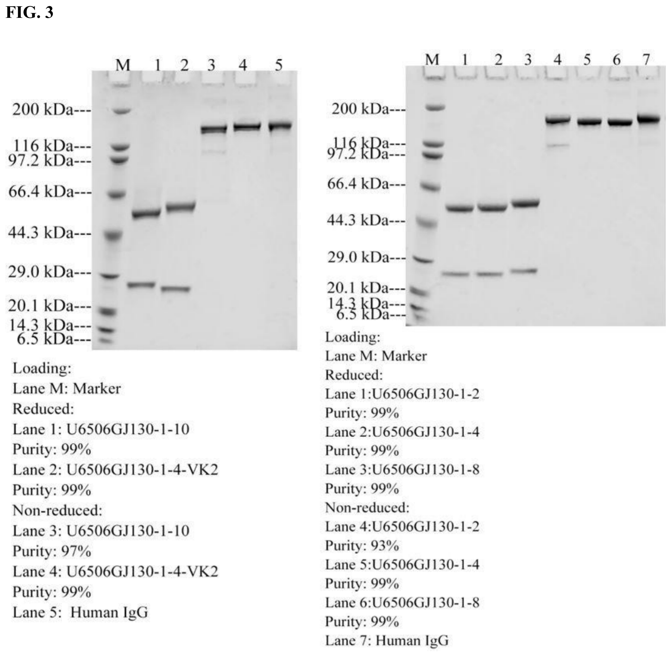

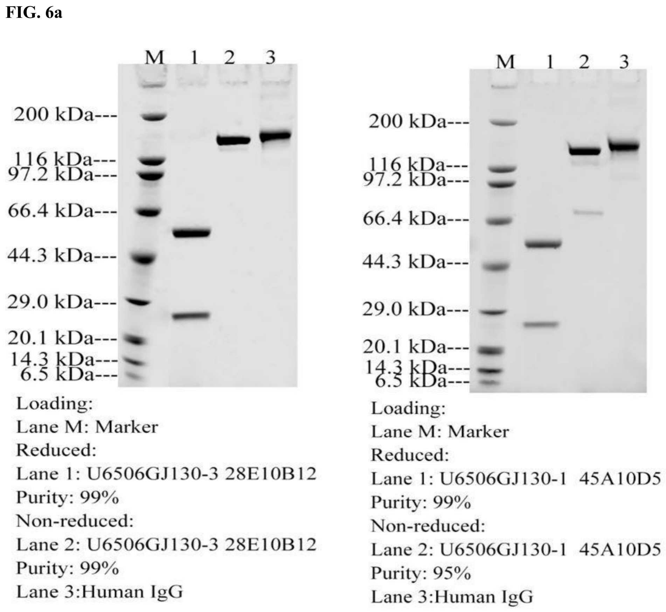

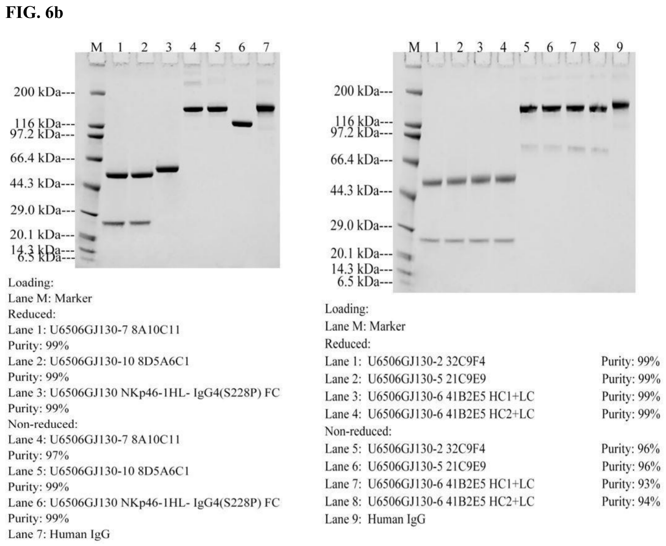

The candidate antibodies were recombinantly expressed and purified as chimeric anti-human NKp46 antibodies that combine the variable region of a mouse-derived monoclonal antibody with the constant region of a human antibody. 3µg of purified anti-human NKp46 antibody was separated on SDS-PAGE and the protein was stained with Coomassie brilliant blue solution to confirm the size and purity of the protein (FIGS. 3 and 6). The sizes of the candidate antibodies were confirmed to be in the form of antibody dimers of approximately 150 kDa under non-reducing conditions, and light and heavy chain antibodies of approximately 25 and 50 kDa, respectively, under reducing conditions.

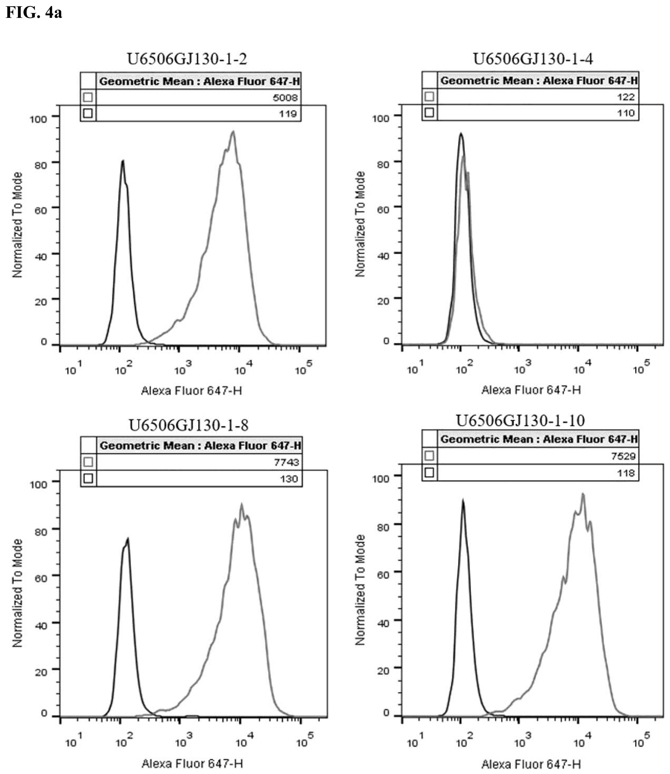

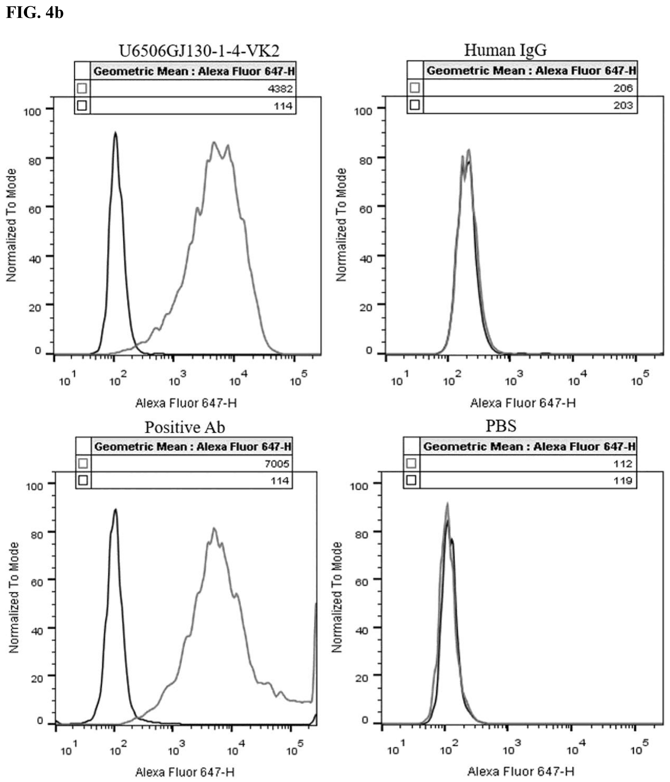

Specifically, human NKp46-expressing CHO-K1 cells in coculture were subjected to centrifugation to remove the culture medium, and the remaining cells were washed twice with an appropriate amount of PBS. 1 x 10⁵ cells were prepared in each well of a 96-well plate and treated with anti-human NKp46 antibody at a concentration of 10µg/mL, followed by incubation for 40 minutes with agitation. After the end of the reaction, each well was washed twice with 150µL of PBS and then treated with fluorescence-conjugated secondary antibody (Alexa Fluor^{®} 647 AffiniPure Fcγ-specific Gort anti-human IgG (min X Bov, Hrs, Ms Sr Prot) (Jackson, 109-605-098)) at a concentration of 1µg/mL and reacted with agitation for 40 minutes. After the end of the reaction, each well was washed twice with 150µL of PBS followed by addition of 150µL of PBS, and binding to human NKp46 was confirmed by flow cytometry (FIG. 4).

Human NKp46 protein and monkey NKp46 protein were each added at a concentration of 0.5µg/mL to a 96-well plate and reacted for 1 hour at 37°C, followed by three washes with an appropriate volume of washing solution (0.05% Tween20, PBS), and then treated with 150µL of blocking solution (1% BSA, PBS) for 1 hour at 37°C. After washing three times with an appropriate volume of washing solution (0.05% Tween20, PBS), the wells were treated with 3 serial dilutions of 1µg/mL of anti-human NKp46 antibody at the maximum concentration and reacted for 1 hour at 37°C. After the end of the reaction, each well was washed three times with 150µL of wash solution, then treated with horseradish peroxidase (HRP) conjugated secondary antibody (Human IgG (H&L), Rackland,309-103-123) at a concentration of 0.1 µg/mL and reacted at 37°C for 30 minutes, followed by three washes with wash solution. The degree of binding between antigen and antibody was detected with 3',5,5'-tetramethylbenzidine solution. Finally, the reaction was stopped by adding 1M HC1, and the absorbance was measured at 450 nm using an ELISA reader (Thermo Fisher Scientific). Four antibodies (1-2, 1-4-2, 1-8, and 1-10) that specifically bind to human NKp46 and monkey NKp46 were selected by the above two binding tests.

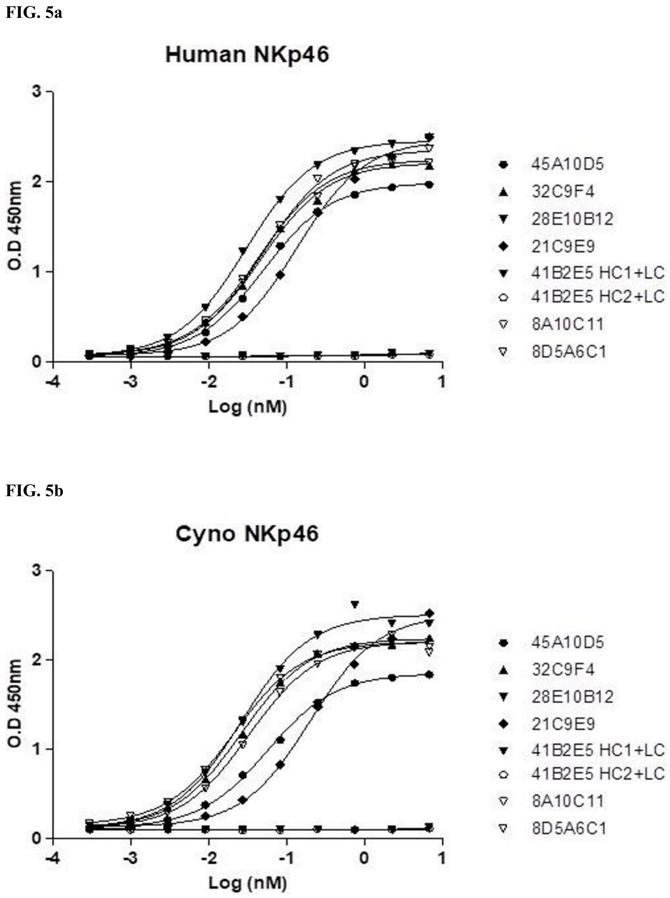

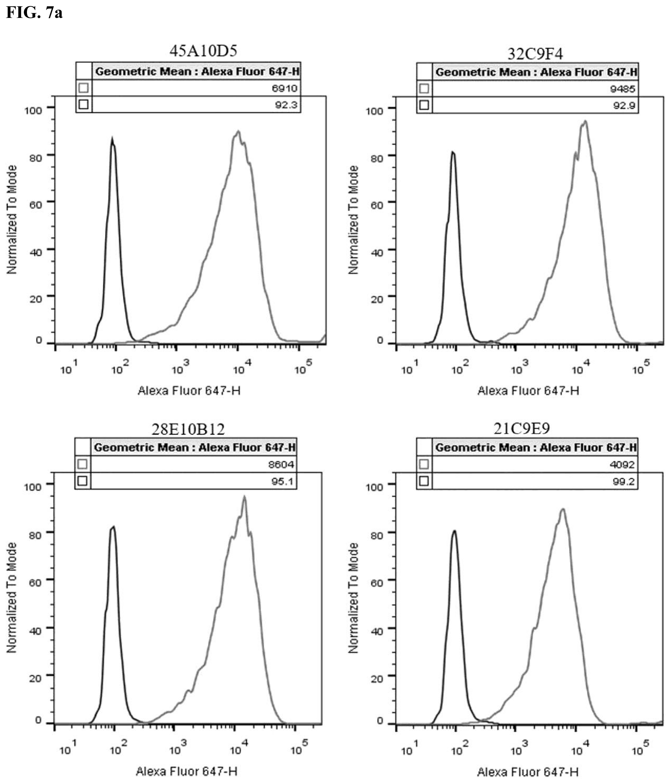

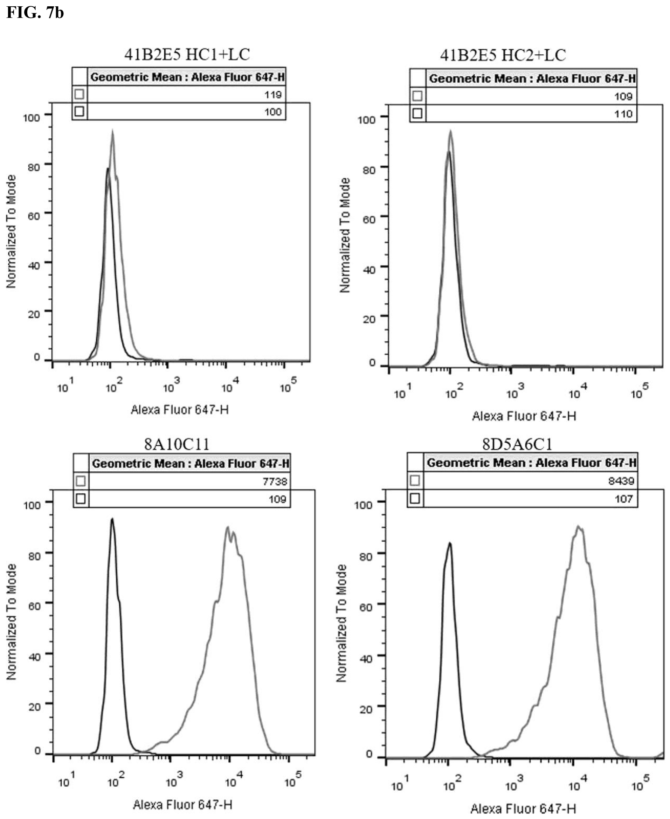

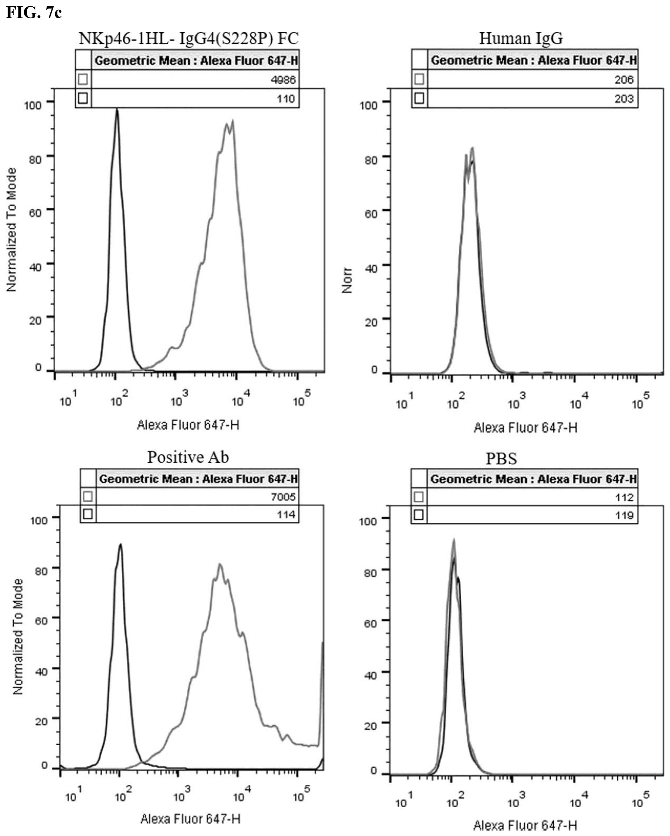

To perform hybridoma screening as another screening method, hybridoma cells were generated by fusing mouse plasma cells with mouse myeloma cells. Among the mouse antibodies produced in the hybridoma cells, clones that specifically bind to human NKp46 and monkey NKp46 were selected and the variable region sequences of the mouse antibodies were analyzed. After production with mouse/human chimeric antibodies, we further screened six antibodies (2-1, 2-2, 2-3, 2-5, 2-7, and 2-10) that specifically bind to human NKp46 and monkey NKp46 by ELISA analysis (FIG. 5) and flow cytometry (FIG. 7).

Through the evaluation of binding capacity and epitope identification of the 10 antibodies obtained by the above two methods, the present inventors selected three antibodies (1-2, 2-3, and 2-5) with the best binding capacity among the antibodies that bind to the same epitope, and then changed the variable regions of the mouse antibodies except for the CDR site to humanized sequences to produce the final three humanized antibodies (Hu 1-2, Hu 2-3, and Hu 2-5).

### Example 2: Measurement of affinity

### Experimental Methods

The Biacore 8K was used to evaluate the affinity of each antibody for its target antigen. The samples, equipment, and reagents used are summarized in Tables 1 and 2 below.

**[Table 1]**

| Samples | MW (kDa) | Concentration (mg/ml) |
|---|---|---|
| 6506GJ130-1A: Human NKP46 Recombinant Protein (His) | 36 | 1 |
| u6506gj 130-1-2 (**1-2**) | 150 | 0.423 |
| u6506gj130-1-8 (**1-8**) | 150 | 0.64 |
| u6506gj 130-1-10 (**1-10**) | 150 | 2.133 |
| u6506gj 130-1-4-vk2 (**1-4-2**) | 150 | 1.711 |
| 45A10D5 (**2-1**) | 150 | 0.38 |
| 32C9F4 (**2-2**) | 150 | 0.487 |
| 28E10B12 (**2-3**) | 150 | 1.425 |
| 21C9E9 (2-5) | 150 | 0.59 |
| 8A10C11 (2-7) | 150 | 1.071 |
| 8D5A6C1 (2-10) | 150 | 0.679 |

**[Table 2]**

| Name | Cat. no. | Lot. No. | Purchase |
|---|---|---|---|
| Biacore 8K | N/A | N/A | Cytiva |
| Series S Sensor Chip Protein A | 29-1275-55 | 10319450 | Cytiva |
| HBS-EP+ Buffer | BR-1006-69 | 32765 | Cytiva |
| Regeneration Buffer: 10 mM Glycine-HCl pH 1.5 | 20220427 | 20220427 | Cytiva |

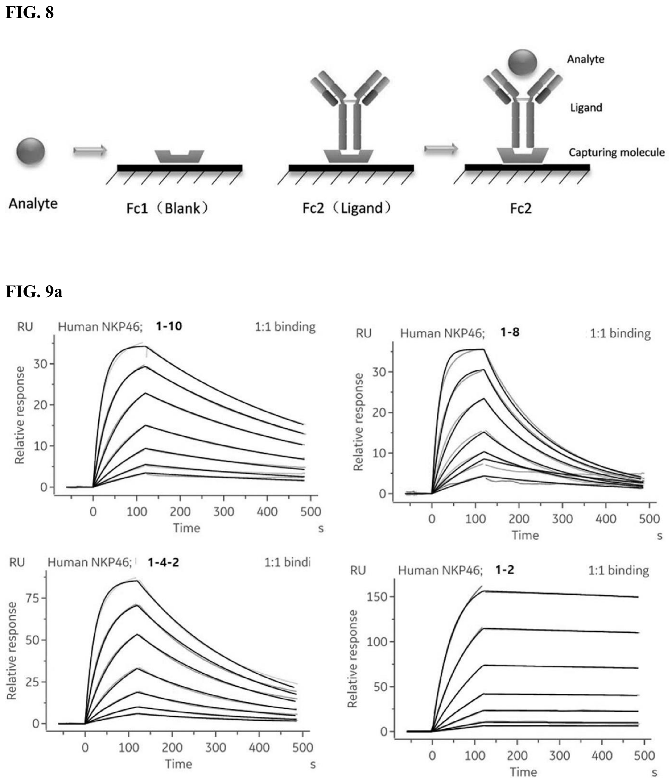

Running buffer was prepared by diluting 10 x buffer with 9x volume of degassed, filtered MilliQ water. Regeneration buffer (10 mM glycine) was prepared by dissolving an aliquot of glycine in MilliQ water and adjusting the pH to 1.5-1.7. Tests were performed at 25°C using HBS-EP+ running buffer. Antibodies were injected for capture on Series S Sensor Chip Protein A. In the binding step, antigens were diluted to various concentrations and injected onto the surface of flow cells 1 and 2, followed by running buffer in the separation step. The binding schematic is shown in FIG. 8, and the running parameters are summarized in Table 3 below.

**[Table 3]**

| Capture | |
|---|---|
| Ligands | Antibodies |
| Capture time (s) | 30 |

| Combine and separate | |
|---|---|
| Mating contact time (s) | 120 |
| Separation contact time (s) | 360 |
| Flow rate (µl/min) | 30 |
| Sample concentration (nM) | 50, 25, 12.5, 6.25, 3.125, 1.5625, 0.78125 (32C9F4/28E10B12/8D5A6C1/8A10C11 to human NKp46) |
| | 100, 50, 25, 12.5, 6.25, 3.125, 1.5625 (U6506GJ130-1-8/45A10D5 to human NKp46) |
| | 200, 100, 50, 25, 12.5, 6.25, 3.125 (U6506GJ130-1-4-VK2/ U6506GJ130-1-10 to human NKp46) |
| | 800, 400, 200, 100, 50, 25, 12.5 (U6506GJ130-1-2/ 21C9E9 to human NKp46) |

| Surface Regeneration | |
|---|---|
| Contact time (s) | 30 |
| Flow rate (µl/min) | 30 |
| Regeneration buffer | 10 mM Glycine-HCl pH 1.5 |

### Result

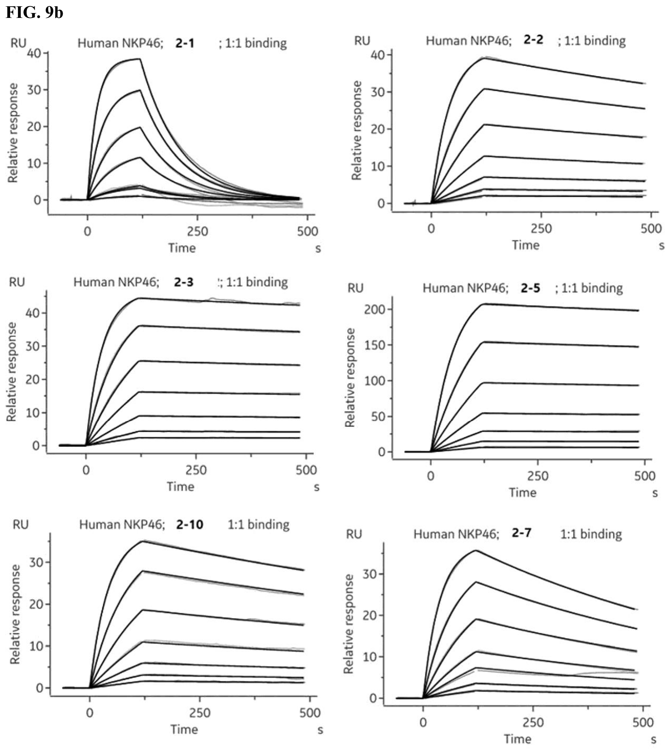

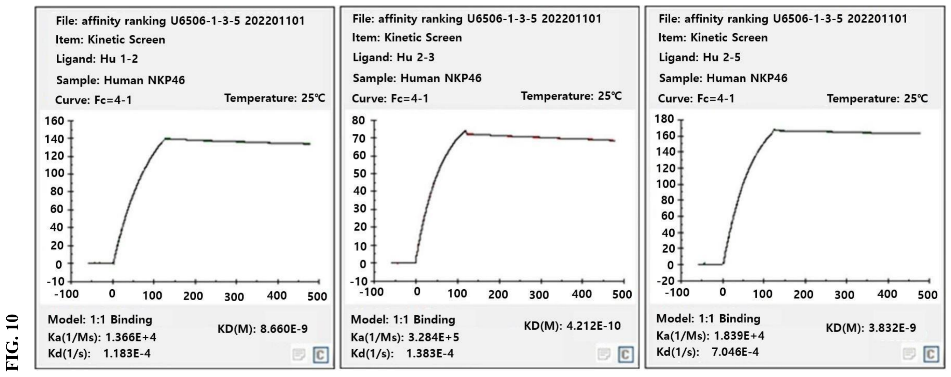

All data were processed using Biacore 8K Evaluation software ver 3.0. In each cycle, a blank injection of flow cell 1 and buffer was used as a double reference for subtraction of response units (RUs). As shown in FIGs. 9a and 9b, it was found that the binding levels of the 10 antibodies of the present invention to human NKp46 were in the range of 0.24-30 nM based on KD. It was also found that the binding levels of the humanized antibodies Hu 1-2, Hu 2-3 and Hu 2-5 to human NKp46 were in the range of 0.42-8.7 nM based on KD (FIG. 10). The three humanized antibodies did not change their KD values by more than 2-fold compared to the unhumanized ones. The calculated affinities of each antibody are summarized in Table 4 below.

**[Table 4]**

| **Ab** | **Antigen** | **Chi² (RU²)** | **ka (1/Ms)** | **kd (1/s)** | **KD (M)** | **Rmax (RU)** |
|---|---|---|---|---|---|---|
| 1-10 | Human NKp46 | 7.36E-02 | 2.46E+05 | 2.29E-03 | 9.34E-09 | 35.5 |
| 1-8 | Human NKp46 | 4.64E-01 | 7.90E+05 | 8.66E-03 | 1.10E-08 | 37.2 |
| 1-4-2 | Human NKp46 | 5.29E-01 | 2.07E+05 | 3.80E-03 | 1.83E-08 | 94.3 |
| 1-2 | Human NKp46 | 4.78E-01 | 2.79E+04 | 1.20E-04 | 4.29E-09 | 178.8 |
| 2-1 | Human NKp46 | 6.35E-01 | 3.76E+05 | 1.13E-02 | 3.00E-08 | 53.6 |
| 2-2 | Human NKp46 | 2.88E-02 | 5.27E+05 | 5.42E-04 | 1.03E-09 | 41.6 |
| 2-3 | Human NKp46 | 5.50E-02 | 5.55E+05 | 1.36E-04 | 2.44E-10 | 45.9 |
| 2-5 | Human NKp46 | 2.64E-01 | 2.34E+04 | 1.16E-04 | 4.96E-09 | 234.2 |
| 2-10 | Human NKp46 | 2.85E-01 | 4.76E+05 | 6.15E-04 | 1.29E-09 | 37.8 |
| 2-7 | Human NKp46 | 1.08E-01 | 5.11E+05 | 1.42E-03 | 2.78E-09 | 39.1 |
| Hu 1-2 | Human NKp46 | 1.41E-01 | 1.37E+04 | 1.18E-04 | 8.66E-09 | 192.1 |
| Hu 2-3 | Human NKp46 | 2.01E-02 | 3.28E+05 | 1.38E-04 | 4.21E-10 | 84.4 |
| Hu 2-5 | Human NKp46 | 5.69E-02 | 1.84E+04 | 7.05E-05 | 3.83E-09 | 202.8 |

### Example 3: Identification of epitopes

The target antigen, human NKp46 recombinant protein (His), was coated 100 µl/well at a concentration of 0.50 µg/ml, and PBS (pH 7.4) was used as the coating buffer. The antibody of the present invention was used as the detection antibody and streptavin-HRP as the secondary antibody.

**[Table 5]**

| Competitive Ab (Final Con.) | 2-1 8.0 ng/ml | 2-2 5.0 ng/ml | 2-3 1.5 ng/ml | 2-5 8.0 ng/ml | 2-7 3.0 ng/ml | 2-10 4.0 ng/ml | 1-2 3.0 ng/ml | 1-8 3.5 ng/ml | 1-10 3.0 ng/ml | 1-4-2 5.0 ng/ml |
|---|---|---|---|---|---|---|---|---|---|---|
| Control | 1.634 | 1.338 | 1.380 | 1.312 | 1.409 | 1.266 | 1.543 | 1.450 | 1.604 | 1.440 |
| 2-1, 20µg/ml | 0.097 | 0.241 | 0.649 | 1.283 | 0.204 | 0.245 | 1.237 | 1.404 | 0.121 | 0.764 |
| 2-2, 20ug/ml | 0.087 | 0.093 | 0.160 | 1.278 | 0.088 | 0.154 | 1.232 | 1.426 | 0.086 | 0.829 |
| 2-3, 20ug/ml | 0.093 | 0.092 | 0.105 | 1.266 | 0.146 | 0.091 | 1.281 | 1.349 | 0.092 | 0.785 |
| 2-5, 20µg/ml | 1.322 | 1.197 | 1.425 | 0.201 | 1.236 | 1.130 | 1.463 | 0.197 | 1.444 | 0.852 |
| 2-7, 20ug/ml | 0.077 | 0.098 | 0.200 | 1.333 | 0.093 | 0.123 | 1.153 | 1.385 | 0.080 | 0.686 |
| 2-10, 20µg/ml | 0.100 | 0.096 | 0.173 | 1.302 | 0.106 | 0.083 | 1.236 | 1.337 | 0.097 | 0.691 |
| 1-2, 20µg/ml | 1.119 | 1.054 | 1.235 | 1.298 | 1.102 | 0.998 | 0.100 | 1.615 | 1.041 | 0.100 |
| 1-8, 20µg/ml | 1.605 | 1.370 | 1.430 | 1.295 | 1.399 | 1.304 | 1.632 | 0.111 | 1.707 | 1.244 |
| 1-10 20µg/ml | 0.087 | 0.159 | 0.408 | 1.396 | 0.133 | 0.176 | 1.190 | 1.449 | 0.095 | 0.662 |
| 1-4-2 20µg/ml | 0.658 | 0.931 | 1.095 | 0.612 | 0.637 | 0.724 | 0.178 | 1.175 | 0.895 | 0.082 |

Antibodies targeting epitope 1 were identified as 2-1, 2-2, 2-3, 2-7, 2-10, and 1-10; antibodies targeting epitope 2 were identified as 1-2 and 1-4-2; and antibodies targeting epitope 3 were identified as 2-5 and 1-8.

**[Table 6]**

| The SEQ ID Nos for the amino acid sequence of each antibody. | | | | | | | | |
|---|---|---|---|---|---|---|---|---|
| Ab | SEQ ID NOs (Amino acid) | | | | | | | |
| | LCDR1 | LCDR2 | LCDR3 | Full VL | HCDR1 | HCDR2 | HCDR3 | Full VH |
| 1-2 | 1 | 7 | 13 | 20 | 33 | 39 | 48 | 57 |
| 1-4-2 | 2 | 8 | 14 | 21 | 34 | 40 | 49 | 58 |
| 1-8 | 3 | 9 | 15 | 22 | 35 | 41 | 50 | 59 |
| 1-10 | 4 | 10 | 16 | 23 | 35 | 42 | 51 | 60 |
| 2-1 | 5 | 11 | 17 | 24 | 35 | 42 | 52 | 61 |
| 2-2 | 5 | 11 | 18 | 25 | 35 | 43 | 52 | 62 |
| 2-3 | 5 | 11 | 17 | 26 | 36 | 44 | 53 | 63 |
| 2-5 | 4 | 10 | 19 | 27 | 37 | 45 | 54 | 64 |
| 2-7 | 5 | 11 | 17 | 28 | 38 | 42 | 55 | 65 |
| 2-10 | 6 | 11 | 18 | 29 | 35 | 43 | 52 | 66 |
| Hu 1-2 | 1 | 7 | 13 | 30 | 33 | 46 | 48 | 67 |
| Hu 2-3 | 5 | 12 | 17 | 31 | 36 | 44 | 53 | 68 |
| Hu 2-5 | 4 | 10 | 19 | 32 | 37 | 47 | 56 | 69 |

**[Table 7]**

| The amino acid sequence of the variable region of each antibody. | | | | | |
|---|---|---|---|---|---|
| Ab | Chain | Amino acid sequence **(sequence** #**)** | | | |
| | | Variable Region | CDR1 | CDR2 | CDR3 |
| 1-2 | VH | EVQLQQSGPELVKPGASVKISCQA SGYTFTTDYYLNWVKQSHGKSLE WIGDIDPHNGGTSYNQKFKDKA TLTVDKSSSTAYMELRSLTSEDSA VYYCARFPIYYSGSSPMDYWGQG TSVTVSS (57) | DYYLN (**33**) | DIDPHNGG TSYNQKFK D (39) | FPIYYSGSS PMDY (48) |
| | 1-2 | DIQMTQSPASSLSASVGETVTITCR TNENIFSYLAWYQQKQGKSSPQLL VYNAKTLAEGVPSRFSGSASGTQF SLKINTLQPEDFGSYYCQHHYGTP YTFTGGGTKLEII (20) | RTNENIFSY LA (1) | NAKTLAE (**7**) | QHHYGTPY T (13) |
| | VL | | | | |
| 1-4-2 | VH | DVQLQQSVAELVRPGASVNLSCTA SGFNIKNTYMHWVKQRPEQGLEW IGMIDPANGNTKYAPKFQGKATIT ADTSSNTAYHLSSLTSEDTAIYYCA RWAYDGYYESYFDYWGQGTTLT VSS (58) | NTYMH (**34**) | MIDPANGN TKYAPKFQ G (40) | WAYDGYY ESYFDY (49) |
| | 1-4-2 | SIVMTQSPKFLLVSAGDRVTITCKA SQSVSNDVAWYQQKPGQSPKLLLI YYASNRYTGVPDRFTGSGYGTDFT FTTISTVQAEDLAVYFCHQDYSSPF TFGSGTKLEIK (21) | KASQSVSN DVA (2) | YASNRYT (**8**) | HQDYSSPF T (14) |
| | VL | | | | |
| 1-8 | VH | QVQLQQPGAELVRPGTSVKLSCKA SGYTFTSYWMHWVKQRPGQGLE WIGVIDPSDSYTNYNQKFKGKATL TVDTSSSTAYMQLSSLTSEDSAVY YCARSSSAYYSNYEAYWGQGTLV TVSA (59) | SYWMH (**35**) | VIDPSDSYT NYNQKFK G (41) | SSSAYYSN YEAY (50) |
| | 1-8 | QIVLTQSPAIMSASPGEKVTISCSAS SSVGYMFWYQQKPGSSPKPWIYR TSNLASGVPARFSGSGSGTSYSLTI SSMEAEDAATYYCQQYHYPPMWT FGGGTKLEIK (22) | SASSSVGY MF (3) | RTSNLAS (**9**) | QQYHYPP MWT (15) |
| | VL | | | | |
| 1-10 | VH | QVQQPGAELVKPGASVKLSCKA SGYIFTSYWMHWVKQRPGQGLEW IGMIHPNSGSTNYNEKFKSKATLTV DKSSSTAYMQLSSLTSEDSAVYYC AREGNWARGYFEYWGQGTTLTVS S (60) | SYWMH (**35**) | MIHPNSGS TNYNEKFK S (42) | EGNWARG YFEY (51) |
| | 1-10 | DIKMTQSPSSMYASLERGERVTITC KASQDINSYS WFQQKPGKSPKTLIYRANRLVDGV PSRFSGSGSGQDYSLTISSLEYEDV GMYYCLQYDEFPYTFGGGTKLEIK (23) | KASQDINS YS (4) | RANRLVD (**10**) | LQYDEFPY T (16) |
| | VL | | | | |
| 2-1 | VH | QVQQQPGAELVKPGASVKLSCKA SGYTFTSYWMHWVKQRPGQGLE WIGMIHPNSGSTNYNEKFKSKATL TVDKSSSTAYMQLSSLTSEDSAVY YCVYDGRFAYWGQGTLVTVSA (61) | SYWMH (**35**) | MIHPNSGS TNYNEKFK S (42) | DGRFAY (**52**) |
| | 2-1 | DIQMTQSPSSLSASLGERVSLTCRA SQDIGSSLNWLQQEPDGTIKRLIYA TSSSLDSGVPKRFSGSRSGSDYSL TISSLESEDFVDYYCLQYASSPFTF GSGTKLEIK (24) | RASQDIGS SLN (5) | ATSSLDS (**11**) | LQYASSPF T (17) |
| | VL | | | | |
| 2-2 | VH | QVQQQPGAELVKPGASVKLSCKA SGYTFTSYWMHWVKQRPGQGLE WIGMIHPNSGVTKYNEKFKSKATL TVDKSSSTAYMQLSSLTSEDSAVY FCAGDGRFAYWGQGTLVTVSA (62) | SYWMH (**35**) | MIHPNSGV TKYNEKFK S (43) | DGRFAY (**52**) |
| | 2-2 | DIQMTQSPSSLSASLGERVSLTCRA SQDIGSSLNWLQQEPDGTIKRLIYA TSSSLDSGVPKRFSGSRSGSDSYSL | RASQDIGS SLN (5) | ATSSLDS (**11**) | LQYASSPW T |
| | VL | TISSLESEDFVDYYCLQYASSPWTF GGGTKLEIK (25) | | | (18) |
| 2-3 | VH | QVQQQPGAELVKPGASVKLSCKA SGYTFTSYWMNWVKQRPGQGLE WIGMIHPNSDITNYNEKFKSQATLT VDKSSSTAYMQLSSLTSEDSAVYY CARHGRFDYWGQGTTLTVSS (63) | SYWMN (**36**) | MIHPNSDIT NYNEKFKS (44) | HGRFDY (**53**) |
| | 2-3 | DIQMTQSPSSLSASLGERVTLTCRA SQDIGSSLNWLQQEPDGTIKRLIYA TSSSLDSGVPKRFSGSRSGSDYSL TISSLESEDFVDYYCLQYASSPFTF GSGTKLEIK (26) | RASQDIGS SLN (5) | ATSSLDS (**11**) | LQYASSPF T (17) |
| | VL | | | | |
| 2-5 | VH | DVQLQESGPGLVKTSQSLSLTCSV TGYSITSDSDYWNWIRQFPGNKLE WVGYISYDGSNNYNPSLKNRISITR DTSKNQFFLKLNSVTTEDTATYYC ARLGYPAMDSWGQGTSVTVSS (64) | SDDYWN (**37**) | YISYDGSN NYNPSLKN (45) | LGYPAMDS (**54**) |
| | 2-5 | DIKMTQSPSSMYASLERGERVTITC KASQDINSYLSWFQQKPGKSPKTLI YRANRLVDGVPSRFSGSGSGQDYS LTISSLEFEDLGIYYCLQYDEFPWT FGGGTKLEIK (27) | KASQDINS YS (4) | RANRLVD (**10**) | LQYDEFPW T (19) |
| | VL | | | | |
| 2-7 | VH | QVQQQPGAELVKPGASVKLSKKA SGFTFTNYWMHWVKQRPGQGLE WIGMIHPNSGSTNYNEKFKSKATL TVDKSSSTAYMQLSSLTSEDSAVY YCAGGGRLDYWGQGTTLTVSS (65) | NYWMH (**38**) | MIHPNSGS TNYNEKFK S (42) | GGRLDY (**55**) |
| | 2-7 | DIQMTQSPSSLSASLGERVSLTCRA SQDIGSSLNWLQQEPDGTIKRLIYA TSSSLDSGVPKRFSGSRSGSDYSL TISSLESEDFVDYYCLQYASSPFTF GSGTKLEIK (28) | RASQDIGS SLN (5) | ATSSLDS (**11**) | LQYASSPF T (17) |
| | VL | | | | |
| 2-10 | VH | QVQQQPGAELVKPGASVRLSCKAF GYTFTSYWMHWVKQRPGQGLEWI GMIHPNSGVTKYNEKFKSKATLTV DKSSSTAYMQLSSLTSEDSAVYFC AGDGRFAYWGQGTLVTVSA (66) | SYWMH (**35**) | MIHPNSGV TKYNEKFK S (43) | DGRFAY (**52**) |
| | 2-10 | DIQMTQSPSSLSASLGERVSLTCRA SQDIGNSLNWLQQEPDGTIKRLIYA TSSSLDSGVPKRFSGSRSGSDYSL TISSLESEDFVDYYCLQYASSPWTF GGGTKLEIK (29) | RASQDIGN SLN (6) | ATSSLDS (**11**) | LQYASSPW T (18) |
| | VL | | | | |
| Hu | VH | QVQLVQSGAEVKKPGASVKVSCK ASGYTFTTDYYLNWVRQAPGQGL EWIGDIDPHNAGTSYNQKFKDRVT TVDTSTSTVYMELSSLRSEDTAVY YCARFPIY YSGSSPMDYWGQGTLVTVSS (67) | DYYLN (**33**) | DIDPHNAG TSYNQKFK D (46) | FPIYYSGSS PMDY (48) |
| 1-2 | Hu | DIQMTQSPSSLSASVGDRVTITCRT NENIFSYLAWYQQKPGKAPKLLLI YYNAKTLAEGVPSRFSGSGSGTDF TLTISSLQPEDFATYYCQHHYGTPY TFTGGGTKLEIK (30) | RTNENIFSY LA (1) | NAKTLAE (**7**) | QHHYGTPY T (13) |
| | 1-2 | | | | |
| | VL | | | | |
| Hu | VH | QVQLVQSGAEVKKPGASVKVSCK ASGYTFTSYWMNWVRQAPGQGLE WIGMIHPNSDITNYNEKFKSRVTM TVDTSTSSTVYMELSSLRSEDTAV YYCARHGRFDYWGQGTTVTVSS (68) | SYWMN (**36**) | MIHPNSDIT NYNEKFKS (44) | HGRFDY (**53**) |
| 2-3 | Hu | DIQMTQSPSSLSASVGDRVTITCRA SQDIGSSLNWLQQKPGKAPKRLIY ATSSLDAGVPKRFSGSGSGTDYTL TISSLQPEDFATYYCLQYASSPFTF GGGTKLEIK (31) | RASQDIGS SLN (5) | ATSSLDA (**12**) | LQYASSPF T (17) |
| | 2-3 | | | | |
| | VL | | | | |
| Hu | VH | QVQLQESGPGLVKPSQTLSLTTVS GYSITSDSDYWNWIRQPPKGLEWI GYISYEGSNNYNPSLKNRVTISRDT SKNQFSLKLSSVTAADTAVYYCAR LGYPAMDAWGQGTLVTVSS (69) | SDDYWN **(37)** | YISYEGSN NYNPSLKN (47) | LGYPAMD A (56) |
| 2-5 | Hu | DIQMTQSPSSLSASVGDRVTITCKA SQDINSYSSWFQQKPGKAPKSLIYR ANRLVDGVPSRFSGSGSGTDYTLTI SSLQPEDFATYYCLQYDEFPWTFG GGTKLEIK (32) | KASQDINS YS (4) | RANRLVD (10) | LQYDEFPW T (19) |
| | 2-5 | | | | |
| | VL | | | | |
| Consta nt region (Com mon) | CH | ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRW QEGNVFSCSVMHEALHNHYTQKSLSLSLGK (70) | | | |
| | CL |  (71) | | | |

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. An antibody or an antigen-binding fragment thereof that specifically binds to NKp46 comprising a heavy chain variable region comprising an HCDR1 having the amino acid sequence selected from the group consisting of SEQ ID NOs: 33 to 38; an HCDR2 having the amino acid sequence region selected from the group consisting of SEQ ID NOs: 39 to 47; and an HCDR3 having the amino acid sequence selected from the group consisting of SEQ ID NOs: 48 to 56.

2. The antibody or the antigen-binding fragment thereof of claim 1, further comprising a light chain variable region comprising an LCDR1 having the amino acid sequence selected from the group consisting of SEQ ID NOs: 1 to 6; an LCDR2 having the amino acid sequence selected from the group consisting of sequences SEQ ID NOs: 7 to 12; and LCDR3 having the amino acid sequence selected from the group consisting of SEQ ID NOs: 13 to 19:

3. The antibody or the antigen-binding fragment thereof of claim 2, wherein the antibody or the antigen-binding fragment thereof is selected from the group consisting of (a) to (m) as set forth below:
(a) an antibody or antigen-binding fragment thereof comprising a light chain variable region comprising LCDR1 having the amino acid sequence of SEQ ID NO: 1, LCDR2 having the amino acid sequence of SEQ ID NO: 7, and LCDR3 having the amino acid sequence of SEQ ID NO: 13; and a heavy chain variable region comprising HCDR1 having the amino acid sequence of SEQ ID NO: 33, HCDR2 having the amino acid sequence of SEQ ID NO: 39, and HCDR3 having the amino acid sequence of SEQ ID NO: 48;
(b) an antibody or antigen-binding fragment thereof comprising a light chain variable region comprising LCDR1 having the amino acid sequence of SEQ ID NO: 2, LCDR2 having the amino acid sequence of SEQ ID NO: 8, and LCDR3 having the amino acid sequence of SEQ ID NO: 14; and a heavy chain variable region comprising HCDR1 having the amino acid sequence of SEQ ID NO: 34, HCDR2 having the amino acid sequence of SEQ ID NO: 40, and HCDR3 having the amino acid sequence of SEQ ID NO: 49;
(c) an antibody or antigen-binding fragment thereof comprising a light chain variable region comprising LCDR1 having the amino acid sequence of SEQ ID NO: 3, LCDR2 having the amino acid sequence of SEQ ID NO: 9, and LCDR3 having the amino acid sequence of SEQ ID NO: 15; and a heavy chain variable region comprising HCDR1 having the amino acid sequence of SEQ ID NO: 35, HCDR2 having the amino acid sequence of SEQ ID NO: 41, and HCDR3 having the amino acid sequence of SEQ ID NO: 50;
(d) an antibody or antigen-binding fragment thereof comprising a light chain variable region comprising LCDR1 having the amino acid sequence of SEQ ID NO: 4, LCDR2 having the amino acid sequence of SEQ ID NO: 10, and LCDR3 having the amino acid sequence of SEQ ID NO: 16; and a heavy chain variable region comprising HCDR1 having the amino acid sequence of SEQ ID NO: 35, HCDR2 having the amino acid sequence of SEQ ID NO: 42, and HCDR3 having the amino acid sequence of SEQ ID NO: 51;
(e) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 17; and a heavy chain variable region comprising HCDR1 having the amino acid sequence of SEQ ID NO: 35, HCDR2 having the amino acid sequence of SEQ ID NO: 42, and HCDR3 having the amino acid sequence of SEQ ID NO: 52;
(f) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 18; and a heavy chain variable region comprising HCDR1 having the amino acid sequence of SEQ ID NO: 35, HCDR2 having the amino acid sequence of SEQ ID NO: 43, and HCDR3 having the amino acid sequence of SEQ ID NO: 52;
(g) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 17; and a heavy chain variable region comprising HCDR1 having the amino acid sequence of SEQ ID NO: 36, HCDR2 having the amino acid sequence of SEQ ID NO: 44, and HCDR3 having the amino acid sequence of SEQ ID NO: 53;
(h) an antibody or antigen-binding fragment thereof comprising a light chain variable region comprising LCDR1 having the amino acid sequence of SEQ ID NO: 4, LCDR2 having the amino acid sequence of SEQ ID NO: 10, and LCDR3 having the amino acid sequence of SEQ ID NO: 19; and a heavy chain variable region comprising HCDR1 having the amino acid sequence of SEQ ID NO: 37, HCDR2 having the amino acid sequence of SEQ ID NO: 45, and HCDR3 having the amino acid sequence of SEQ ID NO: 54;
(i) an antibody or antigen-binding fragment thereof comprising a light chain variable region comprising LCDR1 having the amino acid sequence of SEQ ID NO: 5, LCDR2 having the amino acid sequence of SEQ ID NO: 11, and LCDR3 having the amino acid sequence of SEQ ID NO: 17; and a heavy chain variable region comprising HCDR1 having the amino acid sequence of SEQ ID NO: 38, HCDR2 having the amino acid sequence of SEQ ID NO: 42, and HCDR3 having the amino acid sequence of SEQ ID NO: 55;
(j) an antibody or antigen-binding fragment thereof comprising a light chain variable region comprising LCDR1 having the amino acid sequence of SEQ ID NO: 6, LCDR2 having the amino acid sequence of SEQ ID NO: 11, and LCDR3 having the amino acid sequence of SEQ ID NO: 18; and a heavy chain variable region comprising HCDR1 having the amino acid sequence of SEQ ID NO: 35, HCDR2 having the amino acid sequence of SEQ ID NO: 43, and HCDR3 having the amino acid sequence of SEQ ID NO: 52;
(k) an antibody or antigen-binding fragment thereof comprising a light chain variable region comprising LCDR1 having the amino acid sequence of SEQ ID NO: 1, LCDR2 having the amino acid sequence of SEQ ID NO: 7, and LCDR3 having the amino acid sequence of SEQ ID NO: 13; and a heavy chain variable region comprising HCDR1 having the amino acid sequence of SEQ ID NO: 33, HCDR2 having the amino acid sequence of SEQ ID NO: 46, and HCDR3 having the amino acid sequence of SEQ ID NO: 48;
(l) an antibody or antigen-binding fragment thereof comprising a light chain variable region comprising LCDR1 having the amino acid sequence of SEQ ID NO:5, LCDR2 having the amino acid sequence of SEQ ID NO: 12, and LCDR3 having the amino acid sequence of SEQ ID NO:17 of SEQ ID NO: and a heavy chain variable region comprising having the amino acid sequence of SEQ ID NO: 36, HCDR2 having the amino acid sequence of SEQ ID NO: 44, and HCDR3 having the amino acid sequence of SEQ ID NO: 53; and
(m) an antibody or antigen-binding fragment thereof comprising a light chain variable region comprising LCDR1 having the amino acid sequence of SEQ ID NO: 4, LCDR2 having the amino acid sequence of SEQ ID NO: 10, and LCDR3 having the amino acid sequence of SEQ ID NO: 19; and a heavy chain variable region comprising HCDR1 having the amino acid sequence of SEQ ID NO: 37, HCDR2 having the amino acid sequence of SEQ ID NO: 47, and HCDR3 having the amino acid sequence of SEQ ID NO: 56.

4. The antibody or the antigen-binding fragment thereof of claim 3, wherein (a) comprises a light chain variable region having the amino acid sequence of SEQ ID NO: 20 and a heavy chain variable region having the amino acid sequence of SEQ ID NO: 57.

5. The antibody or the antigen-binding fragment thereof of claim 3, wherein (b) comprises a light chain variable region having the amino acid sequence of SEQ ID NO: 21 and a heavy chain variable region having the amino acid sequence of SEQ ID NO: 58.

6. The antibody or the antigen-binding fragment thereof of claim 3, wherein (c) comprises a light chain variable region having the amino acid sequence of SEQ ID NO: 22 and a heavy chain variable region having the amino acid sequence of SEQ ID NO: 59.

7. The antibody or the antigen-binding fragment thereof of claim 3, wherein (d) comprises a light chain variable region having the amino acid sequence of SEQ ID NO: 23 and a heavy chain variable region having the amino acid sequence of SEQ ID NO: 60.

8. The antibody or the antigen-binding fragment thereof of claim 3, wherein (e) comprises a light chain variable region having the amino acid sequence of SEQ ID NO: 24 and a heavy chain variable region having the amino acid sequence of SEQ ID NO: 61.

9. The antibody or the antigen-binding fragment thereof of claim 3, wherein (f) comprises a light chain variable region having the amino acid sequence of SEQ ID NO: 25 and a heavy chain variable region having the amino acid sequence of SEQ ID NO: 62.

10. The antibody or the antigen-binding fragment thereof of claim 3, wherein (g) comprises a light chain variable region having the amino acid sequence of SEQ ID NO: 26 and a heavy chain variable region having the amino acid sequence of SEQ ID NO: 63.

11. The antibody or the antigen-binding fragment thereof of claim 3, wherein (h) comprises a light chain variable region having the amino acid sequence of SEQ ID NO: 27 and a heavy chain variable region having the amino acid sequence of SEQ ID NO: 64.

12. The antibody or the antigen-binding fragment thereof of claim 3, wherein (i) comprises a light chain variable region having the amino acid sequence of SEQ ID NO: 28 and a heavy chain variable region having the amino acid sequence of SEQ ID NO: 65.

13. The antibody or the antigen-binding fragment thereof of claim 3, wherein (j) comprises a light chain variable region having the amino acid sequence of SEQ ID NO: 29 and a heavy chain variable region having the amino acid sequence of SEQ ID NO: 66.

14. The antibody or the antigen-binding fragment thereof of claim 3, wherein (k) comprises a light chain variable region having the amino acid sequence of SEQ ID NO: 30 and a heavy chain variable region having the amino acid sequence of SEQ ID NO: 67.

15. The antibody or the antigen-binding fragment thereof of claim 3, wherein (l) comprises a light chain variable region having the amino acid sequence of SEQ ID NO: 31 and a heavy chain variable region having the amino acid sequence of SEQ ID NO: 68.

16. The antibody or the antigen-binding fragment thereof of claim 3, wherein (m) comprises a light chain variable region having the amino acid sequence of SEQ ID NO: 32 and a heavy chain variable region having the amino acid sequence of SEQ ID NO: 69.

17. A nucleic acid molecule encoding the antibody or the antigen-binding fragment thereof of claim 1.

18. A gene delivery vehicle comprising the nucleic acid molecule of claim 17.

19. A host cell transformed with the gene delivery vehicle of claim 18.

20. A composition for preventing or treating a cancer or an infectious disease comprising, as an active ingredient, the antibody or the antigen-binding fragment thereof of claim 1, the nucleic acid molecule of claim 17, or the gene delivery vehicle of claim 18.

21. The composition of claim 20, further comprising immune cells.

22. A composition for activating immune cells comprising, as an active ingredient, the antibody or the antigen-binding fragment thereof of claim 1, the nucleic acid molecule of claim 17, or the gene delivery vehicle of claim 18.

23. A composition for detecting NKp46 expressing cells comprising the antibody or the antigen-binding fragment thereof of claim 1 as an active ingredient.

24. A composition for diagnosing a cancer comprising the antibody or the antigen-binding fragment thereof of claim 1 as an active ingredient.
